# EUROPEAN PATENT APPLICATION

(11) **EP 0 642 762 A2**
(43) Date of publication of application: **15.03.1995**
(21) Application number: 94117830.3
(22) Date of filing: 11.07.1988
(51) Int. Cl.: A61B 8/12, G10K 11/00

(54) **Ultrasonic probe**

(30) Priority: 13.11.1987 US 120370
(62) Divisional of application: 88306318.2
(71) Applicant: ADVANCED DIAGNOSTIC MEDICAL SYSTEMS, INC., Dublin California 94568 (US)
(72) Inventor: Terwilliger, Richard A., San Ramon, California 94583 (US); Lamson, Thomas D., Milpitas, California 95035 (US)
(74) Representative: Madeley, June Margaret

(57) **Abstract**

An ultrasonic probe 10 includes an elongated housing 12 which contains a window 24 surrounding an ultrasonic transducer 32. The ultrasonic transducer 32 is mounted on a first platform 36. The first platform 36 is in turn mounted on a second platform 38. A motor 44 and linkage arrangement 48 is provided for pivoting the transducer 32. A thumb wheel 16 can rotatably position the transducer 32 as it pivots, allowing ultrasonic imaging of bodily tissue through three hundred and sixty degrees. Further, a position sensor 78 allows for accurate sensing of the position of the transducer 32 as the transducer 32 pivots and rotates.

## Description

The present invention relates to ultrasonic probes and in particular to ultrasonic probes used for human intracavity examination.

One of the several presently available noninvasive techniques for examining humans, as well as other animate and inanimate objects, includes the use of ultrasound to produce ultrasonic images of portions of the human bodily tissues which would otherwise be inaccessible except by surgical techniques. Ultrasound devices generally require the use of probes which can be applied either externally or internally with resect to the body in order to produce the appropriate image. Quite naturally, with probes that must be applied internally, it is important that the probe be made as small as possible so that the probes can be accommodated in the cavity, whether the cavity is that possessed by an infant or a fully grown adult.

An ultrasonic scanning probe is able to sample data so that an image can be made of a cross-sectional slice or plane through the body. Prior devices have been designed as elongated probes which have the capability of either taking an image representing a slice along the length of the probe or transverse to the length of the probe. The image is accomplished by causing an ultrasonic transducer to scan back and forth in the plane of the image. Prior devices have also been able to take an image by use of a fixed ultrasonic transducer which is located at the tip of the probe. Additionally, biplanar devices include both a scanning ultrasonic transducer for taking an image which is transverse to the probe and also a fix ultrasonic transducer for taking an image along an axial or longitudinal plane.

The disadvantage of the first several probes which only allow for presenting an image in one plane, is that often times there is a requirement to present an image in another plane whether that plane be perpendicular to the first plane or at an oblique angle to the first plane. Thus, in order to accomplish this task, multiple probes are required. In the case of the biplanar probe, only two perpendicular views are presented, and no oblique views are possible. Further, it is to be understood that the two ultrasonic transducers in the biplaner probe are not positioned in the same location on the probe. Thus, in order to take images in two directions at the same position in the human body, the probe must be moved so that one and then the second sensor is properly positioned.

Other examples of ultrasound equipment are disclosed in DE-A-3224290 (Siemens AG) and EP-A-0234951 (Cardiovascular Imaging Systems Inc).

DE-A-3224290 discloses mammography equipment which employs ultrasound. The equipment includes an applicator for receiving a breast and an ultrasound transformer which rotates either continuously or stepwise with respect to the applicator opening so as to scan the breast. A correspondingly continuously or stepwise changing image is represented on a viewing monitor of a video processing device. For every azimuthal position of the ultrasound transformer system the imaging angle can be altered by tilting the transformer system.

EP-A-0234951 on the other hand concerns a catheter apparatus which provides for high resolution intravascular imaging to assist indovascular lesions and to monitor the results of interventional therapy. An ultrasonic transducer is carried by the distal end of a catheter adapted for insertion into a vessel. Either the transducer or another element is rotated and/or moved longitudinally relative to the catheter to image different portions of the vessel.

However, it would be desirable to provide an alternative ultrasonic probe which is compact and which can allow for multiple imaging at any desired location.

With probes, and in particular ultrasound probes, in order to send and receive a high quality signal, generally a fluid medium is provided between the ultrasound probe and the element being imaged. For example, when an ultrasound probe is used externally to a patient in order to image tissue in the patient, a gel-type material is applied to the patient's skin with the portion of the probe housing the ultrasound transducer directed towards the tissue, and totally immersed in the gel.

For imaging human tissue with an internally applied ultrasound probe it is common practice to provide a flexible sheath around the probe, which sheath can be selectively filled with ultrasound transmitting fluid. Thus, where a probe is introduced into a human cavity, the sheath can be expanded with fluid so that the sheath conforms to the human cavity, thereby providing a continuum of fluid from the ultrasound transducer in the probe to the human tissue. Should such a continuum not be provided, as for example when for some reason the sheath does not properly expand or where air is entrapped inside the sheath, the ultrasound signal is distorted as it must travel not only through the fluid medium but also through air.

In prior art devices applied internally with a surrounding expandable sheath, it has been found that internal body muscles such as, for example, the sphincter muscle, can contract and restrict the proper flow of fluid through the sheath in order to fully expand the sheath to conform to the body cavity. When this occurs, the probe must be repositioned or withdrawn and repositioned so that the proper amount of fluid can expand the sheath, filling the entire internal cavity.

After an image has been obtained, the inflatable sheath must be drained of its fluid so that the probe can be comfortably removed from the internal cavity. Again, often times this draining process is inhibited by, for example, a sphincter muscle which clamps down on the sheath and prohibits the withdrawal of fluid from the sheath. Additionally, the sheath itself can become drawn into fluid exhaust ports, which allow the fluid obtained by the sheath to be drained. If the sheath is drawn into such exhaust ports, or if the sphincter or a similar muscle clamps down on the sheath, the sheath cannot be drained. Thus in order to extract the probe from the cavity, it must be extracted forcibly resulting in the sheath rupturing as the probe is extracted.

Generally muscles such as the sphincter muscles are located at the entrance to the cavities and once this muscle is passed, the sheath is free to expand without any restrictions placed on it by the muscle. Thus it is desirable to place the fluid outlet port as close as possible to the ultrasound transducer which is placed in the center of the cavity.

With a pivoting or wobbler type transducer, the transducer itself must be mounted generally in a fluid filled cavity defined in the probe which allows the transducer and any associated mechanisms, such as for example linkage mechanisms, to move appropriately in order to cause the transducer to wobble. The transducer wobbles adjacent a window provided in the housing of the probe which is transparent to ultrasound signals. Thus while it is desirable to place the fluid outlet port as close as possible to the transducer, the cavity in which the transducer is mounted prevents this desired placement. This cavity dictates that the port of necessity is positioned where it is possible that the muscle can clamp down and restrict the flow of fluid through the port to the portion of the sheath which must be expanded to fill the internal cavity.

The present invention is directed to improving upon the present state of the art.

According to a first aspect the present invention provides for an ultrasound probe as set out in the accompanying Claim 1.

Preferably the fluid jacket includes an opening defined by an edge, which edge at least partially defines a border around the window. The fluid jacket includes a recess which can communicate the fluid port with the edge of the opening in order to provide fluid directly to the window.

Preferably, the recess is substantially cylindrical in shape such that fluid can be provided to a substantial portion of the edge of the opening that surrounds the window.

Preferably, the fluid jacket is tubular and fits over a cylindrical probe.

Preferably, a port is provided through the fluid jacket in order to aid in the exhaustion of fluids from between a sheath which can be disposed about the probe and the probe itself. The port through the outer jacket is spaced from and not aligned with the fluid port through the probe so that as water is exhausted through the fluid ports, the sheath cannot be drawn into either of the fluid ports and thus prevent full exhaustion of the fluid.

From the above it can be seen that the present invention provides for a fluid jacket surrounding a probe in order to direct fluid to the ultrasound transparent window. With such an arrangement, should the probe be inserted into an internal cavity, even though a cavity muscle such as a sphincter muscle may clamp down on the probe, the water jacket provides for fluid to be communicated between a fluid port in the probe and an opening in the jacket which is adjacent to the window, through which the ultrasound signals are provided. Thus a sheath provided around the probe and fluid jacket would be properly expanded to fill the entire internal cavity, as a muscle would be unable to restrict such flow. During evacuation of the fluid, the muscle will also be unable to restrict the flow.

As indicated above, the port through the probe body is also used to evacuate fluid from the space between the sheath and the probe. During evacuation, the sheath is not drawn into this port, preventing evacuation of the fluid, as the fluid jacket covers this port. The fluid jacket provides for another port which is spaced from and not in line with the probe port. Thus fluid is exhausted through this fluid jacket port and also along the edge adjacent the window. This being the case, the sheath is not drawn in through the various ports and proper evacuation of the fluid is achieved so that the probe can be conveniently removed from the body cavity without rupturing the sheath.

According to other preferred embodiments the present invention includes an ultrasonic probe capable of sending and receiving an ultrasonic signal. The probe includes an ultrasonic transducer and a mechanism for pivotally and rotationally mounting the ultrasonic transducer to a housing. The probe further includes a motor for pivoting the transducer and a device for operably connecting the motor to the mounting mechanism. The probe further includes a mechanism for rotating the transducer while the motor pivots the transducer.

The probe according to these embodiments consequently provides for a transducer which can collect data to describe an image plane, which image plane can be represented on appropriate pictorial mechanisms such as, for example, a video monitor. As is evident from the above, the invention includes means for rotating the image plane so that images from one location on any desired plane can be obtained for the selected tissue under examination.

The present invention may include a linear motor with another transducer or sensor, for sensing the position of the motor. A linkage is provided between the linear motor and the other transducer. The position of the motor and, thus the ultrasonic transducer, is accurately measured by the position transducer. As the ultrasonic transducer is rotated, the relative relationship between the motor and the position of the ultrasonic transducer is not altered and, thus, the position transducer continues to give an accurate measurement of the position of the ultrasonic transducer no matter at what angle the ultrasonic transducer is set to obtain an image.

Accordingly, embodiments of the present invention provide for a compact ultrasonic probe which can provide multiple imaging of human tissue with the probe located in one position relative to the body. In that position, an image representing a slice or plane through the tissue can be obtained at any or all desired angle. These images can be combined by an appropriate computer mechanism to provide a three dimensional image of the desired tissue.

Accordingly, it is desired to provide a compact ultrasonic probe for intracavity examination.

It is further desired to provide a probe such that a single transducer located at one position on the probe can be used to take images representing slices or planes through the tissue at any desired angle without having to move the probe and without having to substitute another probe which has a transducer which allows an image to be taken in the next desired location.

It is also desired to allow the transducer to change positions while the probe is inserted into the bodily cavity.

It is further desired to provide for imaging through three hundred and sixty degrees at one location so that a truly three dimensional sonic image of the appropriate tissue can be reconstructed.

It is further desired to provide for a probe having an ultrasonic transducer which can be positioned at any desired angle and which includes another transducer for indicating the position of the ultrasonic transducer at whatever position the ultrasonic transducer is presently creating an image.

Fig. 1 is a perspective view of an ultrasonic probe depicting the ability of the probe to take images at one location through any desired angle.

Figs. 2, 2A and 2B are side partial sectional views taken longitudinally along the probe of Fig. 1.

Fig. 3 is a plan partial sectional view which is substantially perpendicular to the view in Fig. 2 taken along a longitudinal axis.

Fig. 4 is a perspective view of an embodiment of an ultrasound probe and a fluid jacket of the invention.

Fig. 4A is a cross-sectional view of Figure 4.

Fig. 5 is a partial cross-sectional view of an embodiment of the fluid jacket of the invention in Figure 4.

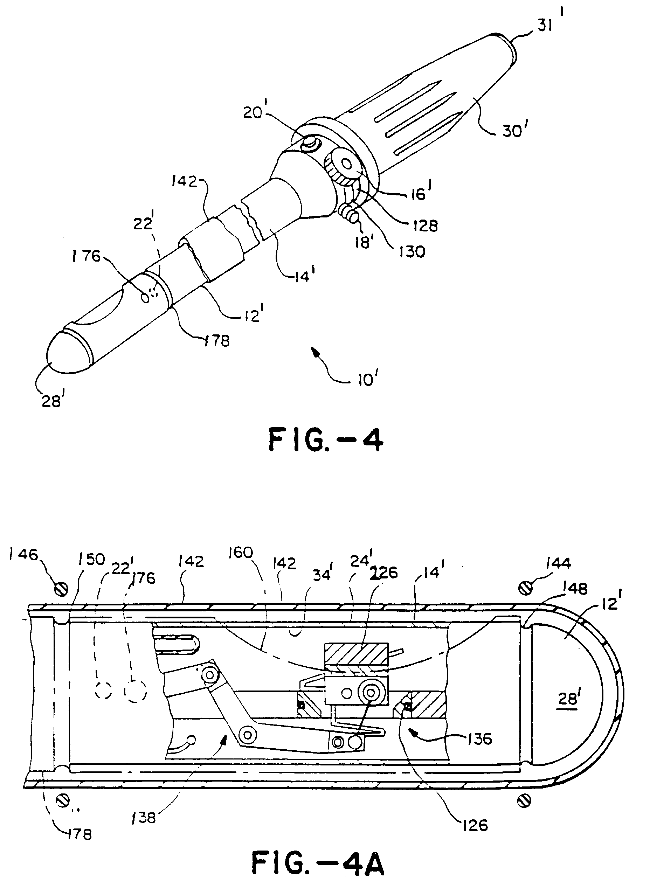

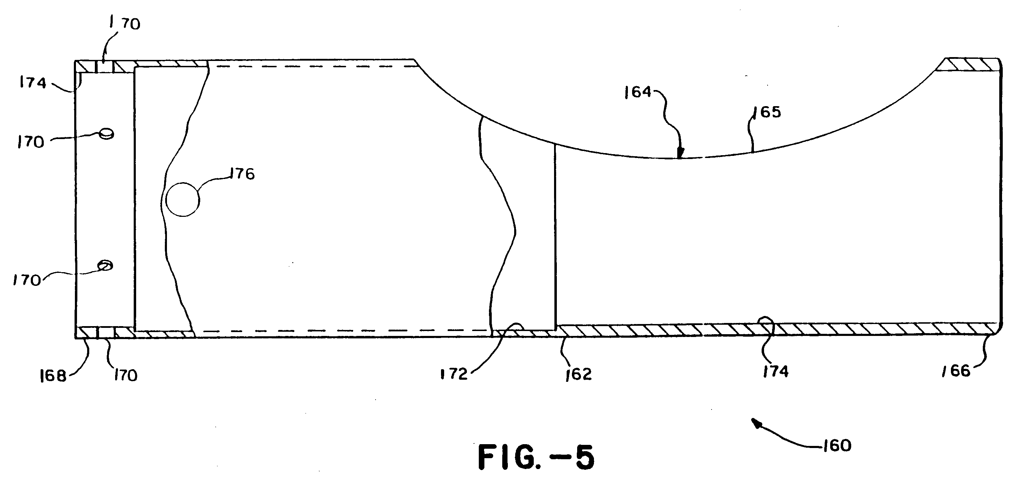

Turning firstly to Figures 4, 4A and 5 an ultrasound probe 10' includes an elongated housing or body 12' which has a cylindrical side surface 14'. The probe includes a thumbwheel 16' for rotationally positioning an ultrasound transducer sensor. The probe 10' further includes introduction and exit ports such as ports 18', 20' for introducing and removing fluid.

The probe 10' further includes an internal water port 22' which is located adjacent to a window 24'.

The window 24' of probe 10' is formed as part of the elongated housing or body 12'. An ultrasound transducer 226 for sending and receiving ultrasound signals is positioned in the probe immediately adjacent the window 24'. The probe further includes a distal end 28' and a handle 30'. A multipin connector (not shown) can be inserted into the end 31' of the handle 30' in order to connect the probe to the appropriate power supply and an appropriate computer device. The computer device provides an electrical signal to the ultrasound transducer and receives from the ultrasound transducer an appropriate response, and from the probe a position signal. With the signal from the transducer and the position signal, the computer device can create an ultrasound image of the tissue being sensed.

The ultrasound transducer 226 which is located adjacent the window 24' is contained in a fluid filled chamber 34'. The chamber allows the transducer 226 sufficient space to enable it to move in all the desired directions and additionally to house the mounts 136 which mount the transducer 226 and also a linkage arrangement 138 which causes the ultrasound transducer 226 to move as desired.

The internal fluid port 22' is as close as possible to the chamber 34' and the window 24' in order to provide, as indicated above, the fluid adjacent to the transducer 226 to fill a sheath 142 with fluid. Properly filled, the sheath can fill an internal body cavity, providing a continuum for the ultrasound signal. The sheath 142 in a preferred embodiment is made out of a latex rubber. The sheath is held in position by bands 144 and 146 which cause the sheath 142 to be urged into grooves 148 and 150 provided in the probe 10'.

An embodiment of the invention includes a fluid jacket 160 which includes a tubular, cylindrical body 162 which is disposed about the window 24' and the cylindrical side surface 14' of the probe 10'. In a preferred embodiment, the jacket is comprised of stainless steel. However, other materials can be used and be within the scope of the invention.

The cylindrical body 162 of the water jacket 160 includes an opening 164 which in a preferred embodiment is substantially elliptical. Other opening configurations can be used as long as the opening accommodates the full range of movements of the transducer. In other words, the opening must be large enough so that the signal sent from or received by the transducer is not blocked or in any way impaired by the fluid jacket 160.

The fluid jacket 160 includes first and second ends 166 and 168. The internal diameter of each of these ends is slightly larger than the external diameter of the cylindrical side surface 14' of the probe 10'. Thus the water jacket 160 can be slid over the cylindrical side surface 14' into a place on the probe 10'. A plurality of set screws 170 are disposed adjacent the second end 168 in order to secure the water jacket 160 to the housing 12' of the probe 10'.

As can be seen in Figure 5, the fluid jacket 160 includes a recessed portion 172 which has an internal diameter which is slightly larger than the internal diameter of the fluid jacket as located at the first and second ends 166, 168. This recessed portion 172 is positioned so that it is located adjacent to and above the internal probe port 22'. Further the recessed portion extends to at least part of the opening 164. As can be seen in Figure 5, the recessed portion 172 is cylindrical and extends completely around the internal cylindrical surface 174 of the fluid jacket 160. It is to be understood that the recessed portion 172 can extend so that it contacts more of the edge 165 of the opening 164. As shown in Figure 5, about a quarter of the edge 165 of the opening 164 is directly communicated with the recessed portion 172. Thus fluid exiting from probe port 22' can communicate through the recessed portion 172 directly with that portion of the edge 165 which communicates with the recessed portion 172.

Fluid jacket 160 includes a port 176 which is provided through cylindrical body 162 at a location which is adjacent to but offset from the internal port 22' of the probe 10' so that the internal port 22' and the fluid jacket port 176 are not aligned. Further the port 176 of the water jacket 160 is provided at a location which communicates with the recess portion 172.

A retainer band 178 is provided in a position which is spaced from the second groove 150 in order to facilitate proper positioning of the band 146 which retains sheath 142.

The operation of the fluid jacket 160 of the invention is as follows.

With the fluid jacket 160 properly positioned over the probe 10' and the sheath 142 and bands 144 and 146 appropriately positioned, ultrasound transmitting fluid such as water can be introduced through the ports such as port 18' to fill a space between the sheath 142 and the housing 12'. After an initial amount of fluid is inserted under the sheath 142, the probe is reoriented so that any remaining air and some fluid is withdrawn from beneath the sheath 142 so that only water remains between the sheath 142 and the probe 10'. This withdrawal of fluid and air is accomplished through the port 176 and finally through port 22'. Once this is accomplished, the probe 10' and the sheath 142 are inserted for example into a bodily cavity. After this is accomplished, further fluid is provided through the port 22' so that the sheath 142 can be fully expanded to conform to the internal bodily cavity with the ultrasound fluid fully filling the space between the probe 10' and the body cavity in order to provide for a continuum for the ultrasound signal. It is to be understood that as the fluid is provided adjacent the window 24', due to the fluid jacket 160, there is no possibility that a bodily muscle can clamp down on the internal port 22' preventing the appropriate filling of the sheath.

When the ultrasound imaging procedure is completed, the fluid jacket 160 allows the water to be evacuated from the space between the probe 10' and the sheath 142 so that the probe 10' can be conveniently removed from the bodily cavity. As the water jacket 160 shields the internal port 22', the sheath 142 will not be drawn into the port 22' so as to block port 22' and prevent further evacuation of fluid. Further as evacuation can occur along the edge 165 of the opening 164 which communicates with the recessed portion 172, more area is available for evacuation and thus the sheath 142 will not be able to prevent this evacuation. Further evacuation occurs through the port 176 of the fluid jacket 160. As fluid flows not only through the port 176 but underneath the port 176 in the space defined by the recessed portion 172 and the probe 10', fluid pressures are such that this port 176 will not be closed by the sheath becoming drawn into the port 176. Thus the fluid can be appropriately evacuated from between the probe 10' and the sheath 142 so that the probe and the sheath can be withdrawn from the bodily cavity without the sheath breaking or tearing and fluid leaking therefrom as the probe is withdrawn.

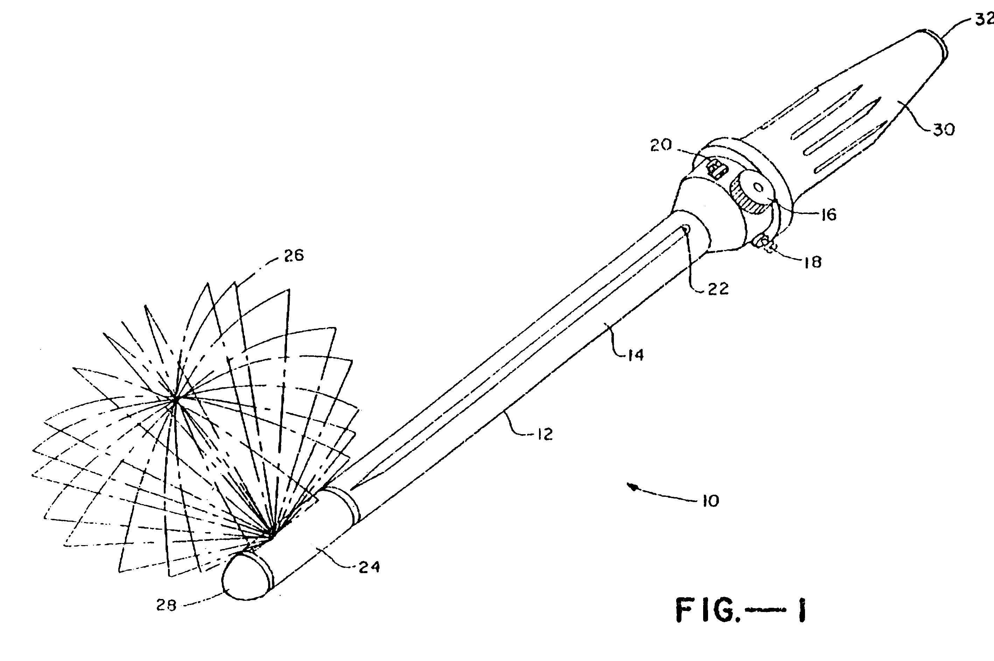

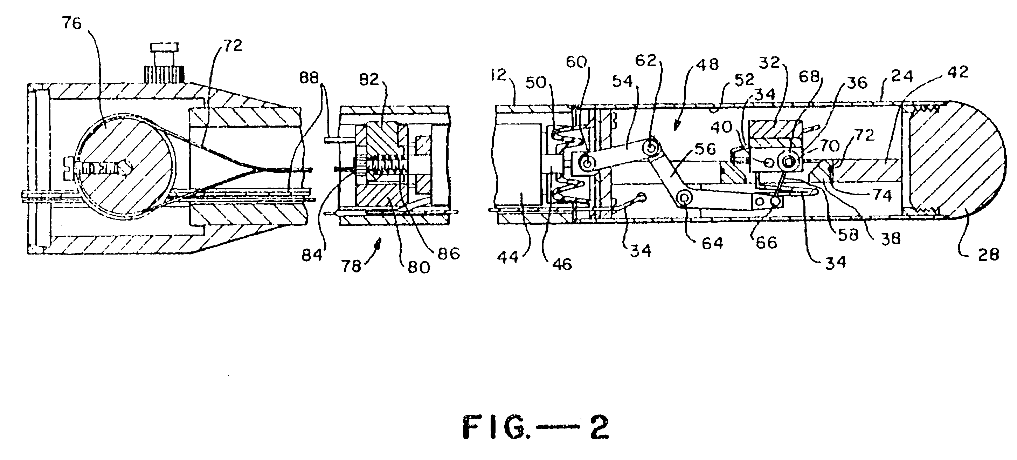

Turning now to Figs. 1 to 3, they illustrate in more detail how a transducer may be mounted in a probe, according to embodiments of the invention.

With respect to these Figs., and in particular to Fig. 1, an ultrasonic probe is depicted and identified by the number 10. As can best be seen in Fig. 1, the probe 10 includes an elongate housing or body 12 which includes a cylindrical side surface 14. The probe 10 includes a thumb wheel 16 for rotationally positioning an ultrasonic transducer or sensor as described below. The probe 10 further includes first and second exterior ports 18, 20 and two interior ports such as interior port 22 for the introduction of fluid as will be described below.

The ultrasonic probe 10 includes a window 24 formed as part of the elongated housing or body 12. As will be described below, an ultrasonic transducer is positioned in the probe immediately below the window 24 in order to send and receive ultrasonic signals as depicted in phantom at 26. The probe includes a distal end 28 and a handle 30. A multipin connector (not shown) can be inserted into the end 32 of handle 30 in order to connect the probe to the appropriate power supply and an appropriate computer device. The computer device provides an electrical signal to the ultrasonic transducer and receives from the ultrasonic transducer an appropriate response, and from the probe, a position signal. With the signal from the transducer and the position signal, the computer device can create an ultrasonic image of the tissue being sensed.

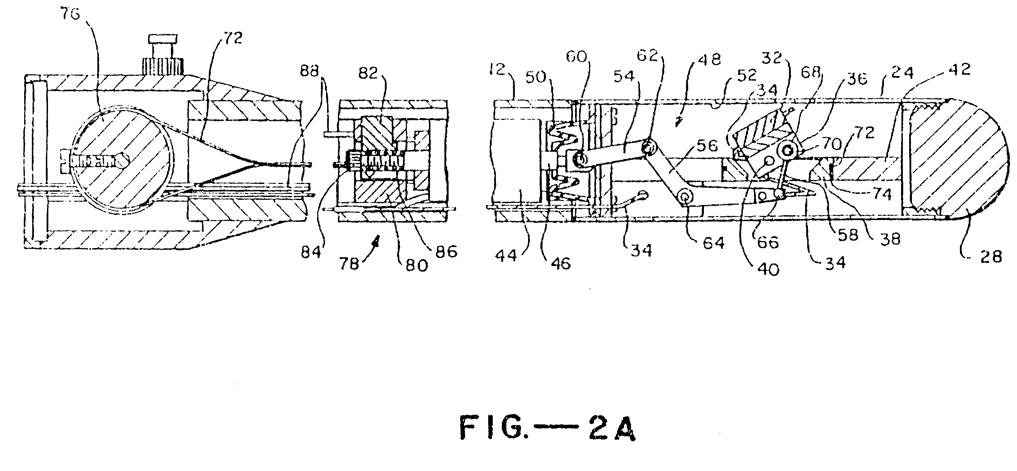

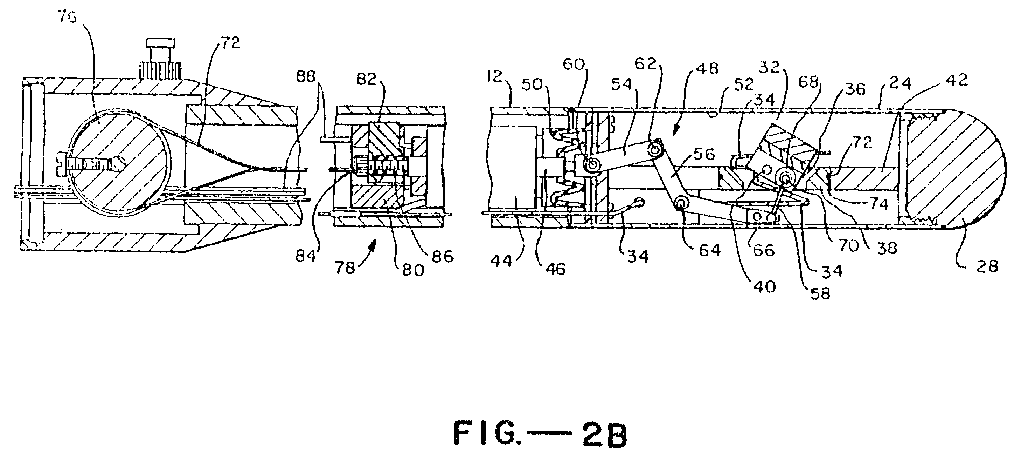

In Figs. 2, 2A and 2B an ultrasonic transducer 32 is depicted. Transducer 32 is connected by way of lead 34 to the above indicated computer device. The ultrasonic transducer 32 is capable of alternatively sending and receiving ultrasonic signals and converting same to and from electrical pulses which are sent on lead 34. Transducer 32 is mounted on a first platform 36. First platform 36 is pivotally mounted on a second platform 38 and is allowed to pivot about a longitudinal pivot axis 40.

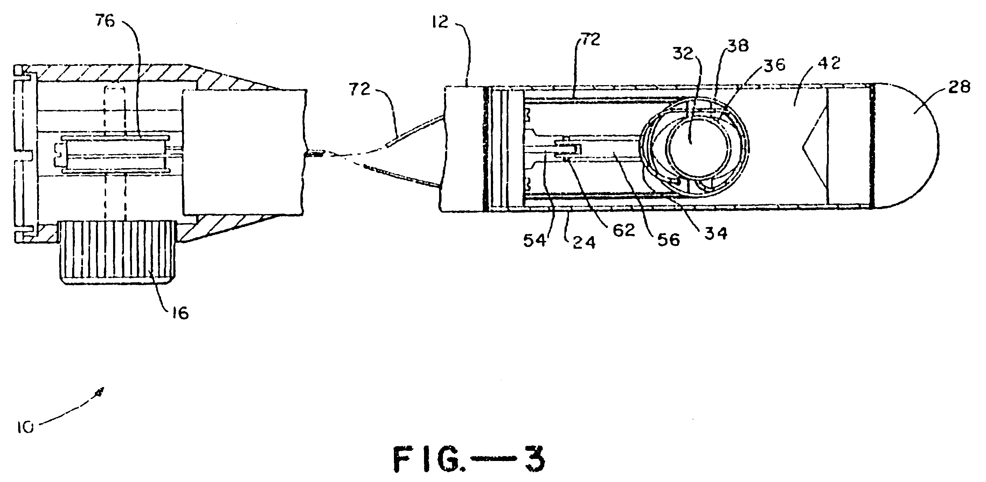

The second platform 38 is rotationally mounted in ultrasonic transducer housing 42 such that second platform 38 can rotate about an axis which is perpendicular to the plane of Fig. 3 Housing 42 is rigidly secured to the elongated body or house 12. In a preferred embodiment, the housing 12 is comprised of a suitably durable and cleanable plastic material which is used in medical applications and the window 24 is comprised of an appropriate clear plastic material.

Positioned midway through the elongated housing 12 in a preferred embodiment is linear motor 44. Also in a preferred embodiment the linear 44 oscillates at the rate of 20 Hz. Secured to the shaft 46 of the linear motor is a linkage arrangement 48 which operably connects the linear motor shaft 46 to the first platform 36 upon which the transducer 32 is mounted. Located immediately adjacent the linear motor 44 is a bellows seal 50 which is connected between the shaft 46 and the housing 12. The bellows seal 50, the cylindrical window 24 and the distal end 28 define a cavity 52 where the linkage arrangement 48, the first and second platform 36, 38 and the ultrasonic transducer 32 are located. In the preferred embodiment, this cavity 52 is filled with a fluid medium, preferably an oil, in order to enhance the transmission of ultrasonic signals to and from the transducer 32.

The linkage arrangement 48 includes a link arm 54, a crank arm 56 and a link rod 58. The link arm 54 is pivotally secured, at pivot point 60, to the shaft 46 of the linear motor 44. In a preferred embodiment, a jewelled pivot arrangement is provided at this location and also at the other pivot points in the linkage arrangement 48 in order to provide for a highly reliable and repeatable accurate mechanism for positioning the transducer 32 responsive to the position of the shaft 46 of the linear motor 44. The link arm 54 is pivotally secured to the crank arm 56 at pivot point 62. Crank arm 56 is pivotally secured at a midpoint 64 to the transducer housing 42. The distal end of crank arm 56 is secured by a ball and socket arrangement 66 to link rod 58. In turn, link rod 58 is secured to a ball and socket arrangement 68, which ball and socket arrangement 68 is itself pivotally mounted to first platform 36 along pivotally axis 70.

Second platform 38 is caused to rotate by a wire 72 that is secured in an outer circumvential groove 74 and which extends to a cable wheel 76 and is secured thereto. Cablewheel 76 is caused to turn by thumb wheel 16. It is to be understood that thumbwheel 16 could be replaced by an appropriate motor in order to motorize the rotation of the second platform 38. Thumb wheel 16 can cause second platform 38 to rotate through one hundred and eighty degrees so that the transducer 32, as transducer 32 scans back and forth, can take an image through three hundred and sixty degrees of rotation.

In order to sense the position of the shaft 46 of the linear motor 44 and thus the position of the transducer 32, a position sensor or transducer 78 is secured to the opposite end of the shaft 46 of the linear motor 44. The position sensor includes a toroidal magnet assembly 80 which in a preferred embodiment is made out of an iron composition and an encoder fin 82 which is secured to the shaft 46 by an appropriate screw 84 and compression spring 86 to ensure the accurate positioning of the fin 82 with respect to the shaft 46. In a preferred embodiment the fin 82 is comprised of aluminum. As the shaft 46 reciprocates, the fin 82 moves back and forth with respect to the toroidal assembly 80. As the encoder fin 82 moves relative to the toroid assembly 80, the inductance of the position sensor 78 changes and this change is sensed by lead 88 which communicates with the computer device (not shown). An appropriate sensing device, such as a potentiometer could be secured to the thumb wheel 16 in order, if desired, to provide the position of the thumb wheel to the computer device.

A sterile sheathing (not shown), preferably a pliable rubber type material, can be secured over the housing 12 before the housing is positioned in the body cavity. The sheathing is secured between the interior port 22 and the exterior ports 18 and 20. Through the exterior ports fluid may be introduced between the sheathing and the housing 12 in order to increase the transfer of ultrasonic signals to and from the transducer 32.

In a preferred embodiment, as previously indicated, the linear motor 44 causes the transducer 32 through the linkage arrangement 48 to pivot at about 20 Hz about pivotal axis 40. The pivoting can be maintained through a complete rotation of the transducer 32 as previously explained. The position sensor 78 can accurately sense the position of the transducer 32 as transducer 32 is pivoted. This position is proportionately related to the change in inductance of the position sensor 78. This proportionality does not change as the transducer 32 is rotated by the thumb wheel 16 and, thus the position of the transducer 32 can be ascertained accurately at all times.

Further, in a preferred embodiment, the transducer can give a resolution of between 200 and 400 lines per 2.5 cm (1 inch) and take a desired image which is approximately 1/40,000 of an inch thick through the appropriate tissue.

From the above it is evident that the present invention has the advantage of being able to accurately and repeatedly position an ultrasonic transducer. Multiple images can be taken so that a three dimensional representation of the tissue can be presented on a computer screen. Further, as can be seen from the above, the present invention is compact, having only one ultrasonic transducer which has all the freedom of movement required in order to be able to select the appropriate image or images required to properly inspect the tissue.

It is to be understood, that while reference is made to using the probe 10 with human tissue, that probe 10 can work equally well with other types of tissue and materials whether living or not which can be inspected with ultrasound.

## Claims

1. A probe (10) having a probe housing (12) and a transducer (32) capable of sending and receiving ultrasonic signals; means (36, 38) for pivotally and rotationally mounting the transducer (32) relative to said housing (12);
motor means (44) for pivoting said transducer (32) so that the signal of the transducer (32) describes a signal plane as the transducer (32) pivots;
means (48) linking said motor means (44) to said pivotally mounting means (36); and
means (16) for rotating said rotationally mounting means (38) while said motor means (44) pivots said transducer (32) such that said signal plane can be caused to rotate./(Fig. 2).

2. A probe according to Claim 1 in which the probe housing (12) is elongate and has an elongate side surface (14) and said means (36) for pivotally mounting said transducer (32) relative to said housing (12) are arranged along said elongate side surface (14) such that the signals can be directed in a plane that is substantially perpendicular to said elongate side surface (14).

3. A probe according to Claim 1 or 2 wherein said pivotally and rotationally mounting means include:
a transducer housing (42) secured inside said probe housing (12);
a first platform (38) rotatably mounted in said transducer housing (42) and a second platform (36) pivotally mounted to said first platform; and
wherein said transducer (32) is secured to said second platform (36).

4. A probe according to Claim 3 wherein the linking means (48) includes:
a link arm (54) pivotally secured to said motor means (44)'
a crank arm (56) pivotally secured to said link arm (54) and pivotally secured to said transducer housing (42);
a shaft means (58) for operably connecting said crank arm (56) to said second platform (36);
means for securing said shaft means (58) to said crank arm (56) such that said shaft (58) can move along any combination of three perpendicular directions;
means for securing said shaft means (58) to said second platform (36) at a location offset from where said second platform (36) is pivotally secured to said first platform (38) such that said shaft (58) can move along any combination of three perpendicular directions.

5. A probe (10) according to any one of claims 1 to 4 wherein the probe housing (12) includes a handle (30) located distally from said transducer (32) and wherein said rotating means (16) includes a pulley (76) mounted in said handle (30) and a cable (72) secured to said first platform (38) and to said pulley (76) for rotating said first platform (38) relative to said transducer housing (42) responsive to the rotation of said pulley (76).

6. A probe according to any of claims 1 to 5 including a sensor means (78) for sensing the position of said motor (44) in order to determine the position of said transducer (32).

7. A probe according to Claim 6 wherein said sensor means (78) includes a toroid magnet (80), a fin (82) and means (84, 86) for securing the fin (82) to the motor means (44) for positioning the fin (82) relative to the toroid magnet (80).
